# EUROPEAN PATENT APPLICATION

(11) **EP 4 775 261 A1**
(43) Date of publication of application: **15.07.2026**
(21) Application number: 24938080.9
(22) Date of filing: 23.12.2024
(51) Int. Cl.: A61N 7/00, A61B 5/00

(54) **ULTRASONIC TRANSDUCER MODULE CONTROL DEVICE HAVING ULTRASONIC FOCAL POINT INDICATOR AND DRIVING METHOD THEREOF**

(30) Priority: 20.11.2024 KR 20240165974
(71) Applicant: Neumous Inc., Cheongju-si, Chungcheongbuk-do 28160 (KR)
(72) Inventor: PARK, Juyoung, Seongnam-si, Gyeonggi-do 13643 (KR); PARK, Chanyuk, Seoul 03668 (KR)
(74) Representative: Schmitt-Nilson Schraud Waibel Wohlfrom Patentanwälte Partnerschaft mbB
(86) International application number: PCT/KR2024/020934
(87) International publication number: WO 2026/111017

(57) **Abstract**

An ultrasonic transducer module control device including an ultrasonic focal point indicator according to an embodiment of the present disclosure may include an ultrasonic transducer module that outputs ultrasound, the ultrasonic focal point indicator manufactured depending on an ultrasonic output field characteristic of the ultrasonic transducer module, and a three-dimensional (3D) movement device for adjusting a location of the ultrasonic transducer module for image registration or location registration.

## Description

### (TECHNICAL FIELD]

The present disclosure relates to an ultrasonic transducer module control device including an ultrasonic focal point indicator and a driving method thereof.

The present disclosure is derived from research conducted as part of Drug Delivery Therapy Technology Development Project of the Ministry of Health and Welfare (Project Unique No.: 2460000920, Project No.: KH135060, Research Project Name:Development of Attachable Ultrasound System Combined with Drug Carrier for Brain Drug Delivery, Project Management Agency: Korea Health Industry Development Institute, Project Implementing Agency: Neumous Co., Ltd., Research Period: 2023.04.01 to 2027.12.31, Contribution Rate: 33.3%), Startup Growth Technology R&D Project of the Ministry of Small and Medium Business and Startups (Project Unique No.: 2420001222, Project No.: 00261874, Research Project Name: Development of Patient-specific Multi-channel Focused Ultrasound System for Opening Blood-Brain Barrier, Project Management Agency: Korea Small and Medium Business Technology Information Promotion Agency, Project Implementing Agency: Neumous Co., Ltd., Research Period: 2023.06.01 to 2026.05.31, Contribution Rate: 33.3%), and Innovative Medical Device Company Technology Commercialization Support Project (R&D) of the Ministry of Health and Welfare (Project Unique No.: 2460001283, Project No.: 00337463, Research Project Name: Through International Collaboration, Development of Ultrasound Device for BBB Opening with High-sensitivity and Real-time Treatment Monitoring, and Clinical Trial, Project Management Agency: Korea Health Industry Development Institute, Project Implementing Agency: Neumous Co., Ltd., Research Period: 2024.04.01 ~ 2026.12.31, Contribution Rate: 33.3%). Meanwhile, there is no property interest of the Korean government in any aspect of the present disclosure.

### [BACKGROUND ART]

A blood-brain barrier (BBB) refers to a physiological barrier that exists within cerebral blood vessels to separate and protect a brain and a central nervous system. This barrier operates as a separator between nerve cells inside the brain and blood.

The inability of treatments for brain diseases to penetrate deep enough into the brain due to the BBB is a significant problem associated with many neurological and medical challenges. In other words, while serving to separate blood and brain tissues to keep the brain protected and safe, the BBB also makes effective delivery of brain disease therapeutics difficult.

In the meantime, a neuromodulation technology refers to a technology that adjusts or modulates neural activities, and means a neural function is changed by applying electrical or chemical stimulation to the brain, a spinal cord, or a peripheral nervous system.

The neuromodulation technology is often used to treat or manage a variety of neurological diseases, including chronic pain, Parkinson's disease, depression, and epilepsy.

Nowadays, various technologies have been developed and researched to treat various brain diseases by controlling the neuromodulation and the BBB by using an ultrasound technology, but there are still technical limitations.

Accordingly, there is a need for a technology that may not only safely and accurately control the neuromodulation and the BBB by using the ultrasound technology, but may also monitor the entire process to overcome the above-described issues and may perform effective procedures.

### [DETAILED DESCRIPTION OF THE INVENTION]

### [TECHINICAL PROBLEM]

The technical problem to be solved by the present disclosure is to measure and analyze the ultrasonic output field characteristics of each ultrasonic transducer in advance and to manufacture a customized ultrasonic focal point indicator suitable therefor.

Moreover, the technical problem to be solved by the present disclosure is to provide an ultrasonic transducer module control device that enables easy location manipulation of an ultrasonic transducer module in which a manufactured ultrasonic focal point indicator is installed, thereby accurately focusing ultrasonic waves at a desired ultrasonic focal point.

Furthermore, the technical problem to be solved by the present disclosure is to construct a brain imaging coordinate system linked with an ultrasonic transducer module control device to visually identify an ultrasonic focal point at the current location of the ultrasonic transducer module, an ultrasonic targeting point at which ultrasonic waves need to be focused, and location coordinates required for location manipulation of a ultrasonic transducer module.

In addition, the technical problem to be solved by the present disclosure is to perform pre-image registration between an ultrasonic focal point and a predetermined reference point on an object, and location registration between the ultrasonic focal point and a targeting point on the object by using a brain imaging coordinate system linked to the ultrasonic transducer module control device, thereby performing high-precision ultrasonic processing.

### [TECHNICAL SOLUTION]

According to an embodiment of the present disclosure, an ultrasonic transducer module control device including an ultrasonic focal point indicator may include an ultrasonic transducer module that outputs ultrasound, the ultrasonic focal point indicator manufactured depending on an ultrasonic output field characteristic of the ultrasonic transducer module, and a three-dimensional (3D) movement device for adjusting a location of the ultrasonic transducer module for image registration or location registration.

Moreover, according to an embodiment of the present disclosure, the ultrasonic output field characteristic may be obtained based on an ultrasonic field distribution and a specification of the ultrasonic transducer module, and the ultrasonic focal point indicator may display an ultrasonic focal point output from the ultrasonic transducer module.

Furthermore, according to an embodiment of the present disclosure, a 3D location of the ultrasonic focal point may match a 3D location of a predetermined reference point of an object.

Besides, according to an embodiment of the present disclosure, the ultrasonic focal point indicator is installed such that one end may be connected to the ultrasonic transducer module and the other end may be located at or in contact with a predetermined reference point of an object.

In addition, according to an embodiment of the present disclosure, a 3D location of the ultrasonic focal point may match a 3D location of the predetermined reference point of the object, and a 3D location of the other end.

Also, according to an embodiment of the present disclosure, the 3D movement device may move the ultrasonic transducer module in a direction of at least one of an X-axis, a Y-axis, and a Z-axis.

Moreover, according to an embodiment of the present disclosure, the 3D movement device may adjust the location of the ultrasonic transducer module to match a 3D location of an ultrasonic focal point output from the ultrasonic transducer module with a 3D location of a predetermined reference point of an object based on a brain imaging coordinate system for the image registration.

Furthermore, according to an embodiment of the present disclosure, the 3D movement device may adjust the location of the ultrasonic transducer module to match 3D location coordinates of the ultrasonic focal point output from the ultrasonic transducer module with a 3D location of a targeting point of the object based on the brain imaging coordinate system for the location registration.

Besides, according to an embodiment of the present disclosure, a driving method of an ultrasonic transducer module control device including an ultrasonic focal point indicator may include measuring an ultrasonic output field characteristic of an ultrasonic transducer module, manufacturing the ultrasonic focal point indicator based on the ultrasonic output field characteristic, installing the manufactured ultrasonic focal point indicator in the ultrasonic transducer module so as to display an ultrasonic focal point output from the ultrasonic transducer module, and adjusting a location of the ultrasonic transducer module for image registration or location registration.

In addition, according to an embodiment of the present disclosure, the adjusting of the location of the ultrasonic transducer module for the image registration may include calculating 3D location coordinates of the ultrasonic focal point and 3D location coordinates of a predetermined reference point of an object, and adjusting a location of the ultrasonic transducer module to match the 3D location coordinates of the ultrasonic focal point with the 3D location coordinates of the predetermined reference point based on a brain imaging coordinate system.

Also, according to an embodiment of the present disclosure, the adjusting of the location of the ultrasonic transducer module for the location registration may further include calculating the 3D location coordinates of the targeting point of the object, and adjusting the location of the ultrasonic transducer module to match the 3D location coordinates of the ultrasonic focal point with the 3D location coordinates of the targeting point based on the brain imaging coordinate system.

Moreover, according to an embodiment of the present disclosure, a non-transitory computer-readable recording medium recorded with a program for executing the driving method of the ultrasonic transducer module control device including the ultrasonic focal point indicator may be included.

### [ADVANTAGEOUS EFFECTS OF THE INVENTION]

According to an embodiment of the present disclosure, an ultrasonic transducer module control device including an ultrasonic focal point indicator, and a driving method thereof may measure and analyze the ultrasonic output field characteristics of each ultrasonic transducer in advance and may manufacture a customized ultrasonic focal point indicator suitable therefor.

Moreover, according to an embodiment of the present disclosure, an ultrasonic transducer module control device including an ultrasonic focal point indicator, and a driving method thereof may provide an ultrasonic transducer module control device that enables easy location manipulation of an ultrasonic transducer module in which a manufactured ultrasonic focal point indicator is installed, thereby accurately focusing ultrasonic waves at a desired ultrasonic focal point.

Furthermore, according to an embodiment of the present disclosure, an ultrasonic transducer module control device including an ultrasonic focal point indicator, and a driving method thereof may construct a brain imaging coordinate system linked with an ultrasonic transducer module control device to visually identify an ultrasonic focal point at the current location of the ultrasonic transducer module, an ultrasonic targeting point at which ultrasound needs to be focused, and location coordinates required for location manipulation of a ultrasonic transducer module.

Also, according to an embodiment of the present disclosure, an ultrasonic transducer module control device including an ultrasonic focal point indicator, and a driving method thereof may perform pre-image registration between an ultrasonic focal point and a predetermined reference point on an object, and location registration between the ultrasonic focal point and a targeting point on the object by using a brain imaging coordinate system linked to the ultrasonic transducer module control device, thereby performing high-precision ultrasonic processing.

### [DESCRIPTION OF THE DRAWINGS]

FIG. 1 is a drawing of an ultrasonic transducer module control device including an ultrasonic focal point indicator, according to an embodiment of the present disclosure.
FIGS. 2A to 2C are diagrams of an ultrasonic field distribution according to the ultrasonic output field characteristics of an ultrasonic transducer module, according to an embodiment of the present disclosure.
FIG. 3 is a drawing of an ultrasonic transducer module, in which a customized ultrasonic focal point indicator is installed, according to an embodiment of the present disclosure.
FIGS. 4A to 4D are diagrams illustrating a pre-image registration step and a location registration step, according to an embodiment of the present disclosure.
FIG. 5 is a flowchart of a driving method of an ultrasonic transducer module control device including an ultrasonic focal point indicator, according to an embodiment of the present disclosure.

### [BEST MODE]

Hereinafter, various embodiments of the present disclosure will be described in detail with reference to the accompanying drawings such that those skilled in the art to which the present disclosure pertains may readily carry out the present disclosure. The present disclosure may be implemented in various different forms and is not limited to the embodiments described herein.

Components or elements not associated with the detailed description may be omitted to describe the present disclosure clearly, and the same reference numerals refer to the same or similar components throughout this application. Therefore, the reference numerals described above may be used in other drawings as well.

Moreover, the size and thickness of each of the components illustrated in the drawings are shown for convenience of explanation and the present disclosure is not necessarily limited thereto. In the drawing, the thickness may be exaggerated to clearly express various layers and regions.

Besides, an expression "the same" in the description may mean "substantially the same". In other words, it may be the same to an extent that a person with ordinary knowledge may understand that it is the same. Other expressions may also be expressions in each of which "substantially" is omitted.

Furthermore, when a portion "comprises" a component in descriptions, it will be understood that it may further include another component, without excluding other components unless specifically stated otherwise. As used herein, a "~unit" or "~part" may be a unit that processes at least one function or operation and may refer to, for example, a software, FPGA, or hardware component. The function provided by the "~unit" or "~part" may be performed separately by a plurality of components, or it may be integrated with other additional components. The "~unit" or "~part" of this specification may not be necessarily limited to software or hardware, and may be configured to be included in an addressable storage medium, or may be configured to operate one or more processors. Hereinafter, embodiments of the present disclosure will be described in detail with reference to the drawings.

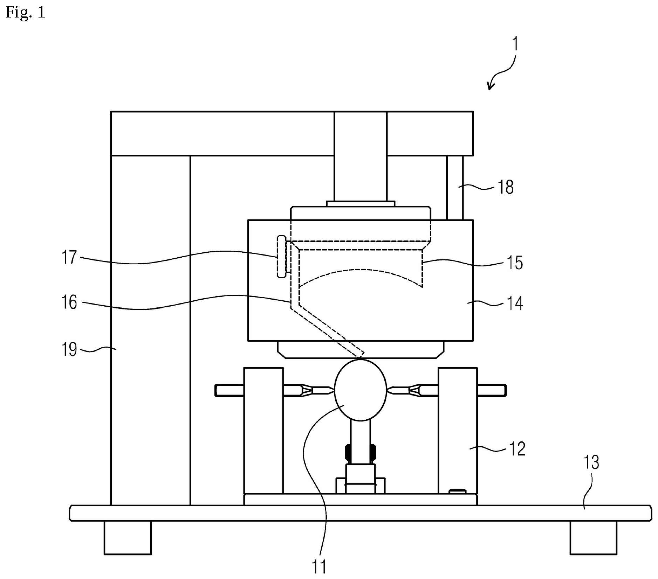

FIG. 1 is a drawing of an ultrasonic transducer module control device including an ultrasonic focal point indicator, according to an embodiment of the present disclosure.

An ultrasonic transducer module control device 1 according to an embodiment of the present disclosure may include a brain fixation device 12, a support plate 13, a chamber 14, an ultrasonic transducer module 15, an ultrasonic focal point indicator 16, a fixation pin 17, an in-chamber air bubble adjustment device 18, and a three-dimensional (3D) movement device 19.

The ultrasonic transducer module control device 1 illustrated in FIG. 1 may be composed of fewer or more components than the components described above.

The brain fixation device 12 may be installed on the support plate 13 and may fix a skull (e.g., brain) of an object 11.

The brain fixation device 12 may be composed of an X-axis direction positioning/orientation module, a Y-axis direction positioning/orientation module, and a Z-axis direction positioning/orientation module. The brain fixation device 12 may restrict movements by fixing the brain of the object 11 located on the support plate 13 in the X-axis, Y-axis, and Z-axis directions.

It is described that the brain fixation device 12 is composed of the X-axis direction positioning/orientation module, the Y-axis direction positioning/orientation module, and the Z-axis direction positioning/orientation module, but the present disclosure is not limited thereto.

The brain fixation device 12 may be composed of two direction (e.g., X and Y axes, Y and Z axes, or Z and X axes) positioning/orientation modules, which may restrict the brain movement of the object 11 in only the corresponding direction.

The support plate 13 may be a plate for supporting the remaining components including the brain fixation device 12. The shape of the support plate 13 may vary.

The chamber 14 may store an acoustic transmission medium. The acoustic transmission medium stored in the chamber 14 may be water. However, as long as any medium facilitates the transmission of ultrasonic waves, it may be used.

In the chamber 14, the ultrasound transmission medium may be injected after or before the brain of the object 11 is fixed. A hole (not shown) for injecting an ultrasound transmission medium may be formed in the chamber 14, and the ultrasound transmission medium may be injected into the interior of the chamber 14 through a hose or the like connected to the hole.

A membrane may be attached or installed at the bottom of the chamber 14. The membrane attached to the chamber 14 may be used to efficiently deliver ultrasound, to accurately receive an acoustic cavitation signal, and to analyze the acoustic cavitation signal.

The membrane attached to the lower part of the chamber 14 may be in contact with the brain of the object 11.

The ultrasound transmission medium may be stored inside the chamber 14, and thus the membrane attached to the chamber 14 may be stretched in the direction of the brain of the object 11 under the influence of gravity. A user or an administrator may contact the brain of the object 11 to a membrane that is attached to the chamber 14 and is stretched.

When the chamber 14 is viewed from the front side, the membrane may be concave and part or all of the brain of the object 11 may be in contact with the membrane, or the membrane may be convex and part or all of the brain of the object 11 may be in contact with the membrane.

The ultrasound output or irradiated from the ultrasonic transducer module 15 may be directed toward the brain of the object 11 through the membrane and the ultrasound transmission medium stored inside the chamber 14.

A through hole may be formed on the lower surface of the membrane attached to the chamber 14. The brain of the object 11 may be inserted through the through hole, part or all of the brain of the object 11 may be exposed inside the chamber 14.

In other words, the acoustic transmission medium stored inside the chamber 14 may be in contact with the exposed brain of the object 11.

Ultrasonic waves output from the ultrasonic transducer module 15 may be transmitted to the brain of the object 11 without interference, by making the acoustic transmission medium stored inside the chamber 14 in contact with the brain of the object 11 through a membrane or directly.

Moreover, the accuracy of analysis may be improved by receiving the acoustic cavitation signal without interference.

The upper surface of the chamber 14 may be provided with the through hole (not shown).

The ultrasonic transducer module 15 may be moved into or out of the chamber 14 through the through hole formed on the upper surface of the chamber 14. The ultrasonic transducer module 15 moved into the interior of the chamber 14 may be in contact with or immersed in the acoustic transmission medium stored inside the chamber 14.

By providing a state where the acoustic transmission medium is in contact with or immersed in the ultrasonic transducer module 15, the ultrasonic waves output from the ultrasonic transducer module 15 may be irradiated to the brain of the object 11 without interference, and the acoustic cavitation signal may be received from the brain of the object 11 without interference, thereby improving the analysis accuracy of the acoustic cavitation signal.

The ultrasonic transducer module 15 may generate ultrasonic waves in the direction of the brain of the object 11 based on a control signal, and may receive the acoustic cavitation signal generated from the brain of the object 11. The detailed structure of the ultrasonic transducer module 15 will be omitted.

The ultrasonic focal point indicator 16 may be one of a variety of devices capable of indicating the location of the ultrasonic focal point output from the ultrasonic transducer module 15.

In detail, as illustrated in FIG. 1, in addition to a device capable of indicating the location of the ultrasonic focal point based on physical contact with the object 11, the ultrasonic focal point indicator 16 may be a device capable of indicating the location of the ultrasonic focal point based on non-physical contact, such as a laser pointer, a magnetic indicator device, a visible light LED pointer, an electrical signal-based indicator device, a virtual reality (VR) or augmented reality (AR) display, an acoustic detection device, and a plasma pointer.

Hereinafter, the ultrasonic focal point indicator 16, which indicates the ultrasonic focal point location based on the physical contact illustrated in FIG. 1, is described as an example.

Referring to FIG. 1, one end of the ultrasonic focal point indicator 16 may be fixed to one side of the ultrasonic transducer module 15 by the fixation pin 17. The other end of the ultrasonic focal point indicator 16 may display the focal point of the ultrasonic waves output from the ultrasonic transducer module 15.

In a state where the other end of the ultrasonic focal point indicator 16 is located or in contact with a predetermined reference point (e.g., Bregma) in the brain of the object 11, the user or the administrator may display the ultrasonic focal point output from the ultrasonic transducer module 15.

In other words, the 3D location of the focal point of the ultrasound output from the ultrasonic transducer module 15 may not only match the 3D location of the predetermined reference point in the brain, but also match the 3D location of the other end of the ultrasonic focal point indicator 16.

The ultrasonic focal point indicator 16 may be located inside the chamber 14.

The ultrasonic focal point indicator 16 may be installed before or after the acoustic transmission medium is introduced into the chamber 14. The other end of the ultrasonic focal point indicator 16 may be installed to be located at or in contact with the predetermined reference point in the brain of the object 11 by manipulation installation of the user or the administrator.

In this case, the 3D location of the ultrasonic focal point and the 3D location of the predetermined reference point in the brain of the object 11 may be displayed on a brain imaging coordinate system displayed on a display module (not shown).

In addition, the 3D location coordinates of the focal point of the ultrasound and the 3D location coordinates of a predetermined reference point in the brain of the object 11 may be displayed together with the brain imaging coordinate system.

The brain imaging coordinate system may be a brain atlas. The brain atlas refers to a map that visualizes the anatomical structure of a brain, and means the functions performed by each part of the brain in a map.

The brain atlas is described as an example, but the present disclosure is not limited thereto. In addition to the brain atlas, various pieces of image data may be used to systematically investigate and visualize structural and functional elements of the brain.

The user or the administrator may visually identify the 3D location and the 3D location coordinates of the ultrasonic focal point, and the 3D location and the 3D location coordinates of a predetermined reference point in the brain of the object 11 through a display module.

In FIG. 1, the ultrasonic focal point indicator 16 is illustrated as having a bent shape at one point, but any shape is possible as long as one end is fixed to one side of the ultrasonic transducer module 15 and the other end is located at or in contact with a predetermined reference point in the brain of the object 11 to display an ultrasonic focal point.

Meanwhile, the ultrasonic focal point indicator 16 may be customized depending on the ultrasonic output field characteristics (pressure field) output by the respective ultrasonic transducer module 15. That is, the specifications of the ultrasonic focal point indicator 16 may vary.

For example, the focal point of the ultrasound may be different depending on the performance of the ultrasonic transducer module 15, and thus the ultrasonic focal point indicator 16 of various specifications suitable for different focal points may be manufactured and provided in advance.

Alternatively, the diameter of the ultrasonic transducer module 15 may be different depending on the performance of the ultrasonic transducer module 15, the ultrasonic focal point indicator 16 of various specifications suitable for the corresponding diameter may be manufactured and provided in advance.

The specifications of the ultrasonic transducer module 15 or the ultrasonic focal point are described as an example, but the present disclosure is not limited thereto.

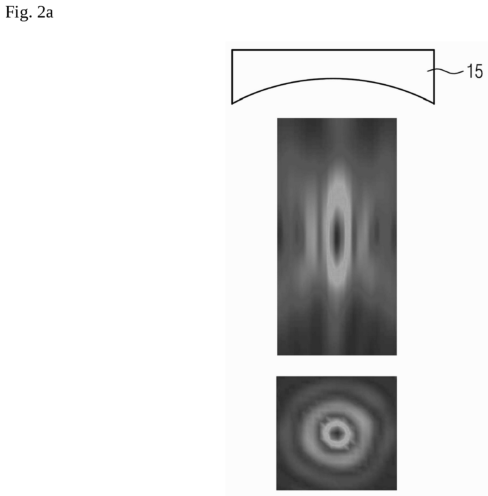

In other words, the ultrasonic focal point indicator 16 of various specifications may be manufactured and provided in advance according to various specifications of the ultrasonic transducer module 15, such as the focal point or specifications. A detailed description will be given later in FIGS. 2A to 2C.

The fixation pin 17 may be used to fix one end of the ultrasonic focal point indicator 16 to one side of the ultrasonic transducer module 15. The location of the ultrasonic focal point indicator 16 may not be adjustable due to the fixation pin 17, or may be adjusted by using the fixation pin 17.

For reference, the ultrasonic focal point indicator 16 is described as being fixed to one side of the ultrasonic transducer module 15 by using the fixation pin 17, but the present disclosure is not limited thereto.

For example, the ultrasonic focal point indicator 16 may be fixed by using the fixation pin 17 to any location of the ultrasonic transducer module 15 where the ultrasonic focal point output from the ultrasonic transducer module 15 may display a predetermined reference point of the object 11.

The in-chamber air bubble adjustment device 18 may remove air bubbles capable of being included in the acoustic transmission medium.

The air bubbles capable of being included in the acoustic transmission medium may adversely affect the output of ultrasonic waves from the ultrasonic transducer module 15, the reception of the acoustic cavitation signal, and the analysis of the acoustic cavitation signal, and thus the in-chamber air bubble adjustment device 18 may be required.

In FIG. 1, the in-chamber air bubble adjustment device 18 is shown as being installed, but the present disclosure is not limited thereto. That is, the in-chamber air bubble adjustment device 18 may be omitted in the ultrasonic transducer module control device 1 according to an embodiment of the present disclosure.

The 3D movement device 19 may adjust the location of the ultrasonic transducer module 15.

In detail, the 3D movement device 19 may be composed of an X-axis direction 3D movement device module, a Y-axis direction 3D movement device module, and a Z-axis direction 3D movement device module.

When it is identified through the display module that the 3D location and the 3D location coordinates (i.e., the 3D location and the 3D location coordinates of an ultrasonic focal point displayed by the ultrasonic focal point indicator 16) of the other end of the ultrasonic focal point indicator 16 do not match the 3D location and the 3D location coordinates of a predetermined reference point in the brain of the object 11, the user or the administrator may allow the 3D movement device 19 to adjust the location of the ultrasonic transducer module 15.

This is a preliminary step for image registration. Under the control of the 3D movement device 19, the 3D location and the 3D location coordinates (i.e., the 3D location and the 3D location coordinates of the ultrasonic focal point indicated by the ultrasonic focal point indicator 16) of the other end of the ultrasonic focal point indicator 16 may change in real time.

When the 3D location and the 3D location coordinates (i.e., the 3D location and the 3D location coordinates of the ultrasonic focal point indicated by the ultrasonic focal point indicator 16) of the other end of the ultrasonic focal point indicator 16 match the 3D location and the 3D location coordinates of a predetermined reference point in the brain of object 11, image registration may be determined to be completed, and the location adjustment of the ultrasonic transducer module 15 may be stopped.

When it is identified through the display module that the 3D location and the 3D location coordinates (i.e., the 3D location and the 3D location coordinates of an ultrasonic focal point displayed by the ultrasonic focal point indicator 16) of the other end of the ultrasonic focal point indicator 16 do not match the 3D location and the 3D location coordinates of a targeting point (e.g., lesion) of the object 11, the user or the administrator may allow the 3D movement device 19 to adjust the location of the ultrasonic transducer module 15.

This is a step for location registration. Under the control of the 3D movement device 19, the 3D location and the 3D location coordinates (i.e., the 3D location and the 3D location coordinates of the ultrasonic focal point indicated by the ultrasonic focal point indicator 16) of the other end of the ultrasonic focal point indicator 16 may change in real time.

When the 3D location and the 3D location coordinates (i.e., the 3D location and the 3D location coordinates of the ultrasonic focal point indicated by the ultrasonic focal point indicator 16) of the other end of the ultrasonic focal point indicator 16 match the 3D location and the 3D location coordinates of the targeting point of the object 11, location registration may be determined to be completed, and the location adjustment of the ultrasonic transducer module 15 may be stopped.

In the meantime, the 3D movement device 19 is described as being manually controlled by the user or the administrator, but the present disclosure is not limited thereto. That is, the 3D movement device 19 may be fully automatically controlled by a separate external driving device.

The ultrasonic transducer module control device 1 may operate in conjunction with an ultrasonic transducer module control system (not shown). The ultrasonic transducer module control system may include a control module (not shown), a display module (not shown), and a database (not shown).

The control module may include an ultrasonic transmitter (not shown) and an ultrasonic receiver (not shown).

The ultrasonic transmitter is used to generate and amplify ultrasonic waves output from the ultrasonic transducer module 15, and may be composed of an ultrasonic signal generator (not shown) and an ultrasonic signal amplifier unit (not shown).

When an ultrasonic control parameter is predetermined by the user or the administrator, the ultrasonic signal generator may generate or produce ultrasonic waves corresponding to the set ultrasonic control parameter. Moreover, the ultrasonic signal amplifier may amplify the corresponding ultrasonic waves.

That is, the ultrasonic transducer module 15 may output or irradiate ultrasonic waves, which are generated and amplified by the ultrasonic signal generator and the ultrasonic signal amplifier, to the brain of the object 11.

Acoustic cavitation-inducing agents (e.g., microbubbles) may be pre-administered to the object 11. The acoustic cavitation-inducing agent administered to the object 11 may move through blood vessels and may be located near or at the targeting point of the object 11.

The ultrasonic waves output or irradiated from the ultrasonic transducer module 15 may be directed toward the targeting point of the object 11 and may be irradiated to the acoustic cavitation-inducing agents located near or at the targeting point.

In this case, the acoustic cavitation-inducing agents may repeat vibration (or oscillation). Moreover, it may generate an acoustic cavitation signal externally.

Meanwhile, the ultrasonic receiver may be used to collect and analyze an acoustic cavitation signal, and may be composed of an ultrasonic signal collector (not shown) and an ultrasonic signal analyzer (not shown).

When an acoustic cavitation signal is generated, the ultrasonic transducer module 15 may receive the corresponding signal and may provide the corresponding signal to the ultrasonic signal collector and the ultrasonic signal analyzer.

The ultrasonic signal analyzer may analyze vibrations of the acoustic cavitation-inducing agents by analyzing the frequency included in the acoustic cavitation signal, and may analyze the degree of BBB control based thereon.

Meanwhile, in the neuromodulation process, the object 11 may not be pre-administered with the acoustic cavitation-inducing agents (e.g., microbubbles). In this case, the energy output or irradiated from the ultrasonic transducer module 15 may be directed to the targeting point of the object 11.

That is, unlike the control process of the BBB described above, in the process of neuromodulation, the vibration (or oscillation) of the acoustic cavitation-inducing agents is not repeated, and thus the analysis process by the acoustic cavitation signal described above may be omitted.

Meanwhile, the present disclosure is described by using BBB control and neuromodulation as an example, but is not limited thereto. This means that any non-invasive treatment utilizing ultrasound may be applied to the technology of the present disclosure.

The display module may be a device that displays a series of information associated with the output of ultrasonic waves and the reception and analysis of the acoustic cavitation signal. That is, it may be a monitor that allows the user or the administrator to visually identify various pieces of information.

The display module may display various pieces of information associated with the brain imaging coordinate system of the object 11 and analysis of the output of ultrasonic waves and the analysis of the acoustic cavitation signal.

That is, the user or the administrator may visually identify various pieces of information displayed on the display module in real time, thereby precisely and easily outputting ultrasonic waves, analyzing the acoustic cavitation signal, and controlling and adjusting the 3D movement device 19 based thereon.

The database may store the brain imaging coordinate system for the object 11 in advance. In detail, by performing various image shootings, such as magnetic resonance imaging (MRI), on the object 11, a brain imaging coordinate system for the object 11 may be obtained.

In this case, data associated with the brain imaging coordinate system of the object 11 may be stored in the database depending on data associated with the respective object 11. Furthermore, identification information for identifying the object 11 may be stored together.

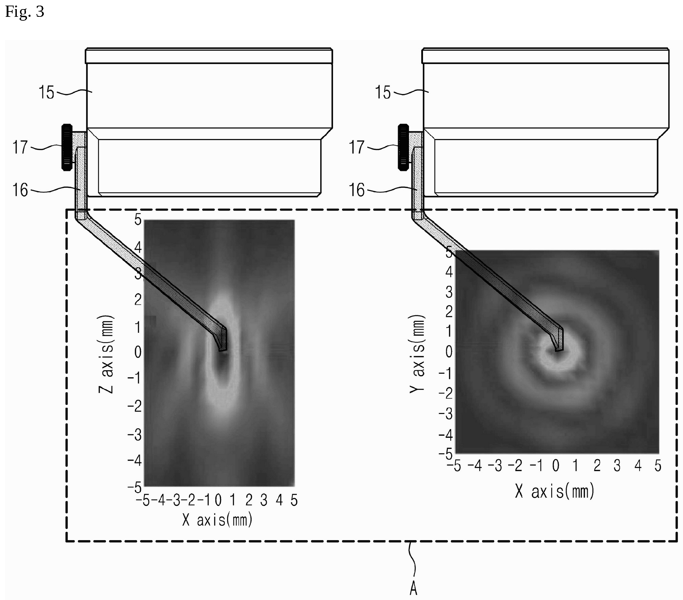

FIGS. 2A to 2C are diagrams of an ultrasonic field distribution according to the ultrasonic output field characteristics of an ultrasonic transducer module, according to an embodiment of the present disclosure. FIG. 3 is a drawing of an ultrasonic transducer module, in which a customized ultrasonic focal point indicator is installed, according to an embodiment of the present disclosure.

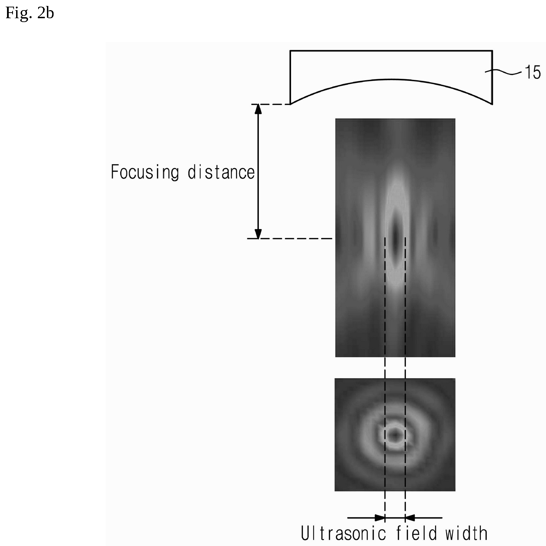

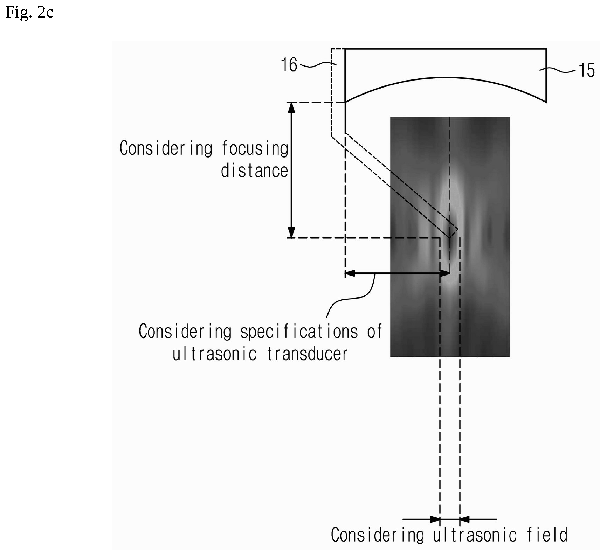

Hereinafter, it will be described with reference to FIGS. 2 and 3 together.

The ultrasonic output field characteristics of the ultrasonic transducer module 15 may vary.

Referring to FIGS. 2A, 2B, and 2C, the ultrasonic output field characteristics of the ultrasonic transducer module 15 may be measured in advance by using an acoustic intensity measurement system (AIMS) technology or a computer simulation technology, but the present disclosure is not limited thereto.

That is, in addition to the AIMS technology or the computer simulation technology, any technology capable of measuring ultrasonic output field characteristics may be utilized.

The control module may analyze various pieces of ultrasonic attribute information (e.g., an ultrasonic field distribution) such as an ultrasonic focusing distance and an ultrasonic field width of the ultrasonic transducer module 15, from the measured ultrasonic output field characteristics of the respective ultrasonic transducer module 15.

The ultrasonic focal point indicator 16 may be pre-manufactured and provided in a plurality of customized ultrasonic focal point indicators in consideration of various pieces of attribute information (e.g., an ultrasonic field distribution), such as the ultrasonic focusing distance and ultrasonic field width of the ultrasonic transducer module 15, as well as the specific specifications of the ultrasonic transducer module 15.

That is, the ultrasonic focal point indicator 16 may be customized in consideration of various pieces of attribute information (e.g., an ultrasonic field distribution), such as the ultrasonic focusing distance and the ultrasonic field width of the ultrasonic transducer module 15, as well as the specifications of the ultrasonic transducer module 15, and thus the 3D location of the ultrasonic focal point indicated by the ultrasonic focal point indicator 16 may precisely match the 3D location of the focal point of the ultrasonic signal actually output from the ultrasonic transducer module 15.

The left side of an image 'A' of FIG. 3 is obtained by visualizing the field distribution of ultrasonic waves output from the ultrasonic transducer module 15, and shows the results measured in the direction of a XZ axis.

In the left side of the image 'A' of FIG. 3, the inside of the white concentric circle represents the focal point where the ultrasonic waves output or irradiated from the ultrasonic transducer module 15 are most concentrated. It moves farther away from the focal point as moving toward the periphery of the concentric circle.

The right side of the image 'A' in FIG. 3 is a photograph obtained by visualizing the field distribution of ultrasonic waves output from the ultrasonic transducer module 15, and shows the results measured in the direction of the XY axis.

In the right side of the image 'A' of FIG. 3, the inside of the white concentric circle represents the focal point where the ultrasonic waves output or irradiated from the ultrasonic transducer module 15 are most concentrated. It moves farther away from the focal point as moving toward the periphery of the concentric circle.

Referring to the left and right sides of image 'A' in FIG. 3, it is identified that the location of the ultrasonic focal point indicated by the ultrasonic focal point indicator 16 precisely matches the location of the focal point within the white concentric circle in the ultrasonic field distribution, which is actually measured in the XZ-axis direction, in the XZ-axis direction.

As described above, the ultrasonic focal point indicator 16 is customized based on the ultrasonic output field characteristics of the ultrasonic transducer module 15 and the specifications of the ultrasonic transducer module 15, and thus the 3D location of the ultrasonic focal point indicated by the ultrasonic focal point indicator 16 may precisely match the 3D location of the ultrasonic focal point actually output from the ultrasonic transducer module 15.

Meanwhile, as described above, any technology capable of measuring ultrasonic output field characteristics may be utilized to measure the ultrasonic output field characteristics of the ultrasonic transducer module 15 in advance.

Accordingly, in addition to a device that displays the ultrasonic focal point location based on a physical contact as shown in FIGS. 2 and 3, the ultrasonic focal point indicator 16 capable of displaying the location of the ultrasonic focal point based on a non-physical contact may also accurately display the 3D location of the ultrasonic focal point.

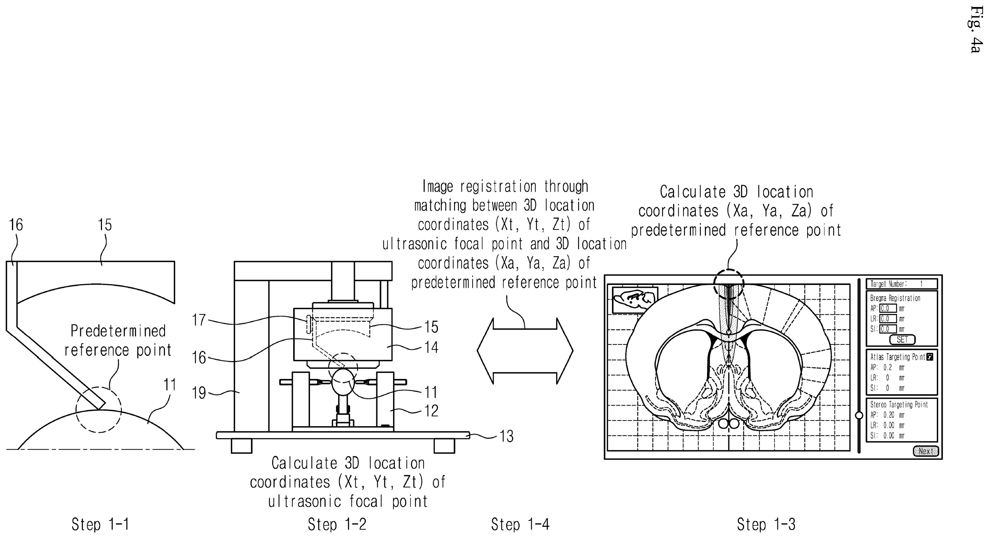

FIGS. 4A to 4D are diagrams of an image registration step and a location registration step, according to an embodiment of the present disclosure.

Hereinafter, it is assumed and described that the 3D location of the other end of the ultrasonic focal point indicator 16 exactly matches the 3D location of the ultrasonic focal point output from the ultrasonic transducer module 15.

Referring to Step 1-1 of FIG. 4A, the location of the predetermined reference point (Bregma) of the object 11 is generally known, and thus a user or an administrator may install the ultrasonic focal point indicator 16 on the side of the ultrasonic transducer module 15 such that the other end of the manufactured ultrasonic focal point indicator 16 is located at or in contact with the predetermined reference point (Bregma) of the object 11 (i.e., such that the location of the ultrasonic focal point indicated by the ultrasonic focal point indicator 16 is located at a predetermined reference point of the object 11).

Referring to Step 1-2 of FIG. 4A, the control module may calculate 3D location coordinates (Xt, Yt, Zt) of the ultrasonic focal point at the current location of the ultrasonic transducer module 15.

In this case, not only the 3D location coordinates (Xt, Yt, Zt) of the ultrasonic focal point, but also the 3D location of the ultrasonic focal point visually may be displayed (e.g., displayed in a region or a point displayed in an arbitrary color) in the brain imaging coordinate system of the display module.

In the meantime, when the location of the ultrasonic transducer module 15 is adjusted, the control module may calculate the 3D location coordinates (Xt, Yt, Zt) of the ultrasonic focal point that change in real time.

In this case, the brain imaging coordinate system of the display module may display not only the 3D location coordinates (Xt, Yt, Zt) of the ultrasonic focal point, which change in real time, but also the 3D location of the ultrasonic focal point visually (e.g., displaying it in a region or a point displayed in an arbitrary color).

Referring to Step 1-3 of FIG. 4A, the control module may calculate 3D location coordinates (Xa, Ya, Za) of a predetermined reference point (Bregma) of the object 11.

In this case, not only the 3D location coordinates (Xa, Ya, Za) of the predetermined reference point of the object 11 calculated by the control module, but also the 3D location of the predetermined reference point of the object 11 may be visually displayed (e.g., displayed in a region or a point displayed in an arbitrary color) in the brain imaging coordinate system of the display module.

In the meantime, it is described that the 3D location coordinates (Xa, Ya, Za) of the predetermined reference point (Bregma) of the object 11 are calculated by the control module, but the present disclosure is not limited thereto.

For example, the 3D location coordinates (Xa, Ya, Za) of the predetermined reference point (Bregma) of the object 11 may be entered by the user or the administrator. Not only the 3D location coordinates (Xa, Ya, Za) of the predetermined reference point (Bregma), but also the 3D location of the predetermined reference point (Bregma) may be visually displayed (e.g., displayed in a region or a point displayed in an arbitrary color) in a display module.

Referring to Step 1-4 of FIG. 4A, whether the 3D location of the ultrasonic focal point matches the 3D location of the predetermined reference point of the object 11 may be determined with reference to the brain imaging coordinate system of a display module.

In detail, the user or the administrator may determine whether the 3D location coordinates (Xt, Yt, Zt) of the ultrasonic focal point displayed in the brain imaging coordinate system of the display module match the 3D location coordinates (Xa, Ya, Za) of the predetermined reference point (Bregma) of the object 11.

Alternatively, the user or the administrator may determine whether the 3D location (e.g., in a region or a point displayed in an arbitrary color) of the ultrasonic focal point visually displayed in the brain imaging coordinate system of the display module matches the 3D location of the predetermined reference point.

When the 3D location and the 3D location coordinates (Xt, Yt, Zt) of the ultrasonic focal point do not match the 3D location and the 3D location coordinates (Xa, Ya, Za) of the predetermined reference point (Bregma) of the object 11, the user or the administrator may adjust the location of the ultrasonic transducer module 15 by controlling the 3D movement device 19.

In other words, the 3D location and the 3D location coordinates of the ultrasonic focal point may match the 3D location and the 3D location coordinates of the predetermined reference point (Bregma) of the object 11 in advance, thereby achieving high-accuracy image registration.

Alternatively, the control module may calculate relative coordinates (Xm, Ym, Zm) between the 3D location coordinates (Xt, Yt, Zt) of the ultrasonic focal point and the 3D location coordinates (Xa, Ya, Za) of a predetermined reference point (Bregma) of the object 11.

The relative coordinates (Xm, Ym, Zm) calculated by a control module may be displayed in the brain imaging coordinate system of a display module. The user or the administrator may allow the 3D movement device 19 by the relative coordinates (Xm, Ym, Zm) to manipulate the location of the ultrasonic transducer module 15, thereby performing image registration.

The present disclosure is described as controlling the 3D movement device 19 by using relative coordinates, but it is not limited thereto.

That is, in addition to the relative coordinates, various image registration algorithms or location correction technologies for image registration may be utilized based on location information of an ultrasonic focal point and location information of the predetermined reference point (Bregma) of the object 11.

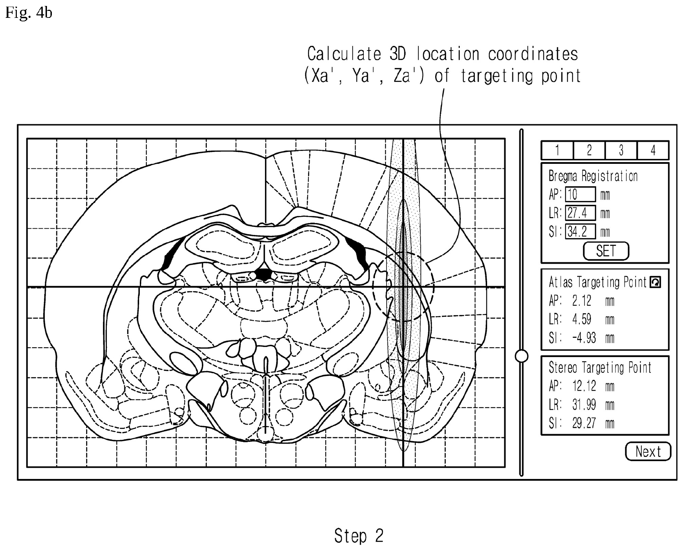

Referring to Step 2 of FIG. 4B, the location of the targeting point where the ultrasound needs to be focused may be visually displayed in the brain imaging coordinate system of the display module.

The control module may calculate 3D location coordinates (Xa', Ya', Za') of the targeting point. The 3D location and the 3D location coordinates (Xa', Ya', Za') of the targeting point calculated by the control module may be displayed in the brain imaging coordinate system.

In detail, data regarding the targeting point may be pre-stored in a database.

The control module may calculate the 3D location coordinates (Xa', Ya', Za') of the targeting point based on data regarding a targeting point of the respective object 11 pre-stored in the database.

The above-described process may be performed directly by the user or the administrator.

For example, when the user or the administrator directly inputs the 3D location coordinates (Xa', Ya', Za') of the targeting point, the display module may visually display the 3D location coordinates (Xa', Ya', Za') of the targeting point and the 3D location of the targeting point in the brain imaging coordinate system.

Alternatively, when the user or the administrator clicks any point corresponding to the targeting point in the brain imaging coordinate system of the display module, the control module may calculate the 3D location coordinates (Xa', Ya', Za') of the targeting point.

As shown in FIG. 4B, the display module may visually display the 3D location and the 3D location coordinates for the targeting point in the brain imaging coordinate system.

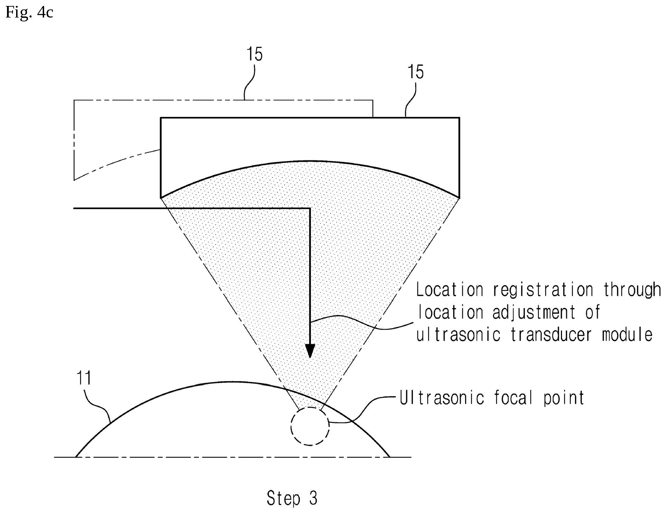

Referring to Step 3 of FIG. 4C, the location of the ultrasonic transducer module 15 may be adjusted to match the 3D coordinates of the ultrasonic focal point and the 3D coordinates of the targeting point.

Hereinafter, it is assumed and described that the 3D location of the ultrasonic focal point output from the ultrasonic transducer module 15 exactly matches the 3D location of the predetermined reference point of the object 11, and thus image registration is completed.

Because the image registration is completed, the ultrasonic focal point indicator 16 installed in the ultrasonic transducer module 15 may be removed.

The user or the administrator may determine whether the 3D location of the ultrasonic focal point output or irradiated from the ultrasonic transducer module 15 matches the 3D location of the targeting point of the object 11, with reference to the brain imaging coordinate system of the display module.

In detail, the user or the administrator may determine whether the 3D location coordinates (Xt, Yt, Zt) of the ultrasonic focal point displayed in the brain imaging coordinate system of the display module match the 3D location coordinates (Xa', Ya', Za') of the targeting point.

Alternatively, the user or the administrator may determine whether the 3D location of the ultrasonic focal point visually displayed in the brain imaging coordinate system of the display module matches the 3D location of the targeting point of the object 11.

When the 3D location and the 3D location coordinates (Xt, Yt, Zt) of the ultrasonic focal point output or irradiated from the ultrasonic transducer module 15 do not match the 3D location and the 3D location coordinates (Xa', Ya', Za') of the targeting point of the object 11, the user or the administrator may adjust the location of the ultrasonic transducer module 15.

In other words, the user or the administrator may accurately match the 3D location of the ultrasonic focal point, which is output or irradiated from the ultrasonic transducer module 15 with reference to the display module, with the 3D location of the targeting point of the object 11.

In this way, high-accuracy location registration between the focal point of the ultrasound and the targeting point of the object 11 may be achieved.

Alternatively, the control module may calculate relative coordinates (Xm', Ym', Zm') between the 3D location coordinates (Xt, Yt, Zt) of the ultrasonic focal point and the 3D location coordinates (Xa', Ya', Za') of the targeting point of the object 11.

The relative coordinates (Xm', Ym', Zm') may be displayed together with the brain imaging coordinate system of the display module. Under the control of the 3D movement device 19, the ultrasonic transducer module 15 may be moved by the relative coordinates (Xm', Ym', Zm'), thereby performing accurate location registration.

It is described that the 3D movement device 19 is manipulated by using the relative coordinates, but the present disclosure is not limited thereto.

That is, in addition to the relative coordinates, various location registration algorithms or location correction technologies may be utilized to match the 3D location information of the ultrasonic focal point with the 3D location information of the targeting point of the object 11.

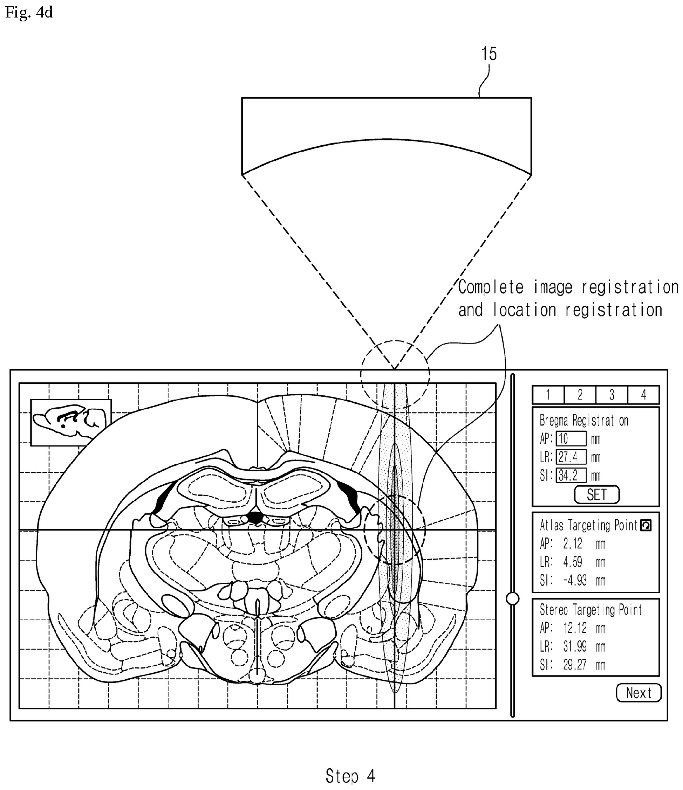

Referring to Step 4 of FIG. 4D, it may be identified that the 3D location and the 3D location coordinates of the ultrasonic focal point exactly match the 3D location and the 3D location coordinates of the targeting point displayed in the brain imaging coordinate system.

In other words, the present disclosure may accurately display the ultrasonic focal point output or irradiated from the ultrasonic transducer module 15 by using the ultrasonic focal point indicator 16, may achieve high-accuracy image registration by matching the 3D location of the ultrasonic focal point with the 3D location of a predetermined reference point of the object 11 in the brain imaging coordinate system, and may achieve high-accuracy location registration by matching the 3D location of the ultrasonic focal point with the 3D location of the targeting point of the object 11 in the brain imaging coordinate system.

In the meantime, the display module may display a button capable of setting an ultrasonic control parameter. The user or the administrator may set the control parameter of the ultrasound in advance by clicking the displayed button.

The display module may display a window showing an acoustic cavitation signal received from the object 11. In this case, the corresponding window may be displayed in the form of a graph with a time axis.

The display module may display a window showing the sonication power of ultrasonic waves output from the ultrasonic transducer module 15. In this case, the corresponding window may be displayed in the form of a graph with a time axis.

In other words, through the display module, ultrasonic control parameters may be easily set, and the entire ultrasonic output and analysis process may also be visually identified and monitored.

The display module may display a report including ultrasonic output and analysis results. The corresponding investigation report may include a series of information related to the output and analysis of ultrasound, and the user or the administrator may receive the process of outputting and analyzing the ultrasound in the form of a report.

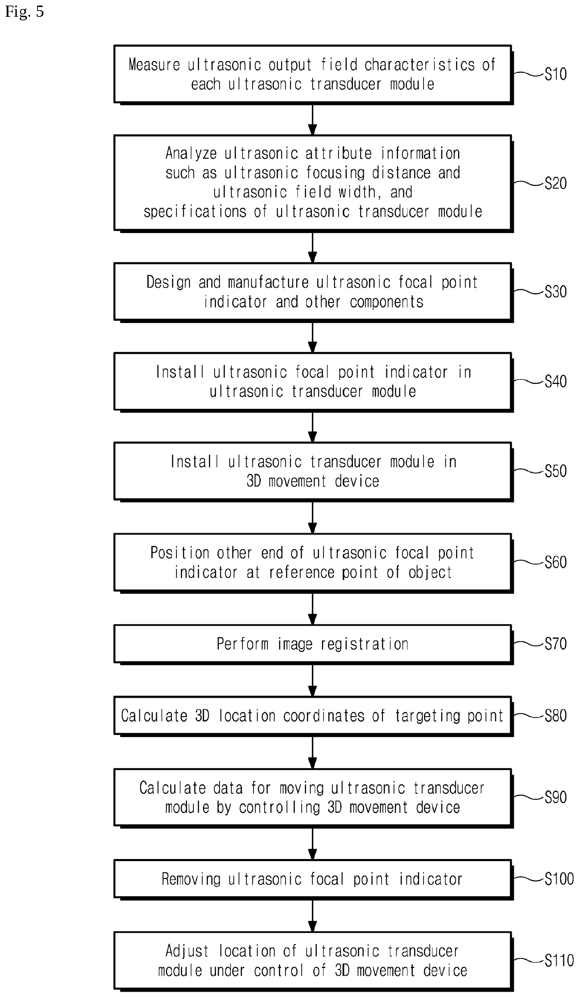

FIG. 5 is a flowchart of a driving method of an ultrasonic transducer module control device including an ultrasonic focal point indicator, according to an embodiment of the present disclosure.

In step S10, the ultrasonic output field characteristics of each ultrasonic transducer module may be measured.

In detail, a user or an administrator may pre-measure the 3D ultrasonic output field characteristics of the respective ultrasonic transducer module 15 by using various technological means for measuring ultrasonic output field characteristics (pressure field), such as an AIMS technology or a computer simulation technology.

In step S20, ultrasonic attribute information such as an ultrasonic focusing distance and an ultrasonic field width, and the specifications of the ultrasonic transducer module may be analyzed.

In detail, the control module may analyze the ultrasonic attribute information such as an ultrasonic focusing distance and an ultrasonic field width of the ultrasonic transducer module 15, from the ultrasonic output field characteristics (pressure field) of the respective ultrasonic transducer module 15.

Information about the specifications of the ultrasonic transducer module 15 may be analyzed separately or by a control module.

In step S30, an ultrasonic focal point indicator and other components may be designed and manufactured.

In detail, the plurality of ultrasonic focal point indicators 16 may be manufactured in a customized manner in consideration of ultrasonic attribute information and the specifications of the ultrasonic transducer module 15.

The ultrasonic focal point indicator 16 is manufactured in a customized manner, and thus the 3D location of the ultrasonic focal point indicated by the ultrasonic focal point indicator 16 may precisely match the 3D location of the focal point of the ultrasonic signal actually output from the ultrasonic transducer module 15.

In step S40, an ultrasonic focal point indicator may be installed in the ultrasonic transducer module.

In detail, one end of the ultrasonic focal point indicator 16 may be fixed to one side of the ultrasonic transducer module 15 via the fixation pin 17.

In step S50, an ultrasonic transducer module may be installed in a 3D movement device.

In particular, one side of the ultrasonic transducer module 15 may be installed to be connected to the 3D movement device 19, and the other side thereof may be installed to be located inside the chamber 14. Moreover, the other components may be assembled.

In step S60, the other end of the ultrasonic focal point indicator may be positioned at a reference point of an object.

In detail, the user or the administrator may install the ultrasonic focal point indicator 16 such that the other end of the ultrasonic focal point indicator 16 contacts or is located at a predetermined reference point in the brain of the object 11 (i.e., such that the location of the ultrasonic focal point indicated by the ultrasonic focal point indicator 16 is located at a predetermined reference point in the brain of the object 11).

In step S70, image registration may be performed.

In particular, the control module may calculate 3D location coordinates of the ultrasonic focal point at the current location of the ultrasonic transducer module 15.

The 3D location and the 3D location coordinates of the ultrasonic focal point output or irradiated from the ultrasonic transducer module 15 may be displayed in the brain imaging coordinate system of the display module.

The user or the administrator may determine whether the 3D location and the 3D location coordinates of the ultrasonic focal point match the 3D location and the 3D location coordinates of the predetermined reference point of the object 11, with reference to the brain imaging coordinate system of the display module.

When the 3D location and the 3D location coordinates of the ultrasonic focal point do not match the 3D location and the 3D location coordinates of the predetermined reference point of the object 11, the location of the ultrasonic transducer module 15 may be adjusted by controlling the 3D movement device 19.

In other words, the 3D location and the 3D location coordinates of the ultrasonic focal point may accurately match the 3D location and the 3D location coordinates of the predetermined reference point (Bregma) of the object 11, thereby performing high-accuracy image registration.

In step S80, the 3D location coordinates of the targeting point may be calculated.

In detail, the control module may calculate the 3D location coordinates of the targeting point of the object 11 where the ultrasound needs to be focused.

The 3D location and the 3D location coordinates of the targeting point of the object 11 may be visually displayed in the brain imaging coordinate system of the display module.

In this case, the user or the administrator may determine whether the 3D location and the 3D location coordinates of the ultrasonic focal point match the 3D location and the 3D location coordinates of the targeting point of the object 11, with reference to the brain imaging coordinate system of the display module.

In step S90, data for moving the ultrasonic transducer module may be calculated by controlling the 3D movement device.

In detail, the control module may calculate data for adjusting the movement of the ultrasonic transducer module 15 from the 3D location and the 3D location coordinates of the ultrasonic focal point and the 3D location and the 3D location coordinates of the targeting point.

In step S100, the ultrasonic focal point indicator 16 may be removed.

In detail, the user or the administrator may remove the ultrasonic focal point indicator 16 installed in the ultrasonic transducer module 15 via the fixation pin 17.

In step S110, the location of the ultrasonic transducer module may be adjusted under the control of the 3D movement device.

In particular, the 3D movement device 19 may be controlled based on the data calculated in step S90.

For example, when the data calculated in step S90 is relative coordinates, the user or the administrator may move the ultrasonic transducer module 15 by the relative coordinates.

In this way, the 3D location and the 3D location coordinates of the ultrasonic focal point may accurately match the 3D location and the 3D location coordinates of the targeting point of the object 11, thereby performing high-accuracy location registration.

The drawings and detailed description of the present disclosure given so far are merely illustrative of the present disclosure. This is only used for the purpose of describing the present disclosure and is not used to limit the scope of the present disclosure described in the meaning or claims. Therefore, it will be understood that various modifications and other equivalent embodiments are possible from this point by those skilled in the art. The technical protection scope of the present disclosure will be defined by the technical spirit of the appended claims.

The above-described embodiments may be implemented with hardware components, software components, and/or a combination of hardware components and software components. For example, the devices, methods, and elements described in the embodiments of the present disclosure may be implemented by using one or more general-use computers or special-purpose computers, such as a processor, a controller, an arithmetic logic unit (ALU), a digital signal processor, a microcomputer, a field programmable gate array (FPGA), a programmable logic unit (PLU), a microprocessor, or any device which may execute instructions and respond.

A processing unit may perform an operating system (OS) or one or software applications running on the OS. Further, the processing device may access, store, manipulate, process and generate data in response to execution of software. It will be understood by those skilled in the art that although a single processing unit may be illustrated for convenience of understanding, the processing unit may include a plurality of processing elements and/or a plurality of types of processing elements.

For example, the processing unit may include a plurality of processors or one processor and one controller. Also, the processing unit may include a different processing configuration, such as a parallel processor. Software may include computer programs, codes, instructions or one or more combinations thereof and configure a processing unit to operate in a desired manner or independently or collectively control the processing unit.

Software and/or data may be embodied in any type of machine, components, physical equipment, virtual equipment, computer storage media or devices so as to be interpreted by the processing unit or to provide instructions or data to the processing unit. Software may be dispersed throughout computer systems connected via networks and be stored or executed in a dispersion manner. Software and data may be recorded in one or more computer-readable storage media.

The methods according to the above-described embodiments may be recorded in a computer-readable medium including program instructions that are executable via various computer devices. The non-transitory computer-readable medium may include program instructions, data files, data structures, etc. independently or may include a combination thereof. The program instructions recorded in the media may be designed and configured specially for the exemplary embodiments of the present disclosure or be known and available to those skilled in computer software.

The computer-readable medium may include a hardware device, which is specially configured to store and execute program instructions, such as magnetic media (e.g., a hard disk drive, a floppy disk, and a magnetic tape), optical media (e.g., CD-ROM and DVD), read only memories (ROMs), random access memories (RAMs), and flash memories. Examples of computer programs include not only machine language codes created by a compiler, but also high-level language codes that are capable of being executed by a computer by using an interpreter or the like. The described hardware devices may be configured to act as one or more software modules to perform the operations of the above-described embodiments, or vice versa.

While embodiments have been shown and described with reference to the accompanying drawings, it will be apparent to those skilled in the art that various modifications and variations may be made from the foregoing descriptions. For example, adequate effects may be achieved even though the foregoing processes and methods are carried out in different order than described above, and/or the aforementioned elements, such as systems, structures, devices, or circuits, are combined or coupled in different forms and modes than as described above or be substituted or switched with other components or equivalents. Therefore, other implements, other embodiments, and equivalents to claims are within the scope of the following claims.

[INDUSTRIAL APPLICABILITY]

## Claims

1. An ultrasonic transducer module control device including an ultrasonic focal point indicator, the device comprising:
an ultrasonic transducer module configured to output ultrasound;
the ultrasonic focal point indicator manufactured depending on an ultrasonic output field characteristic of the ultrasonic transducer module; and
a three-dimensional (3D) movement device for adjusting a location of the ultrasonic transducer module for image registration or location registration.

2. The device of claim 1, wherein the ultrasonic output field characteristic is obtained based on an ultrasonic field distribution and a specification of the ultrasonic transducer module, and
wherein the ultrasonic focal point indicator displays an ultrasonic focal point output from the ultrasonic transducer module.

3. The device of claim 2, wherein a 3D location of the ultrasonic focal point matches a 3D location of a predetermined reference point of an object.

4. The device of claim 2, wherein the ultrasonic focal point indicator is installed such that one end is connected to the ultrasonic transducer module and the other end is located at or in contact with a predetermined reference point of an object.

5. The device of claim 4, wherein a 3D location of the ultrasonic focal point matches a 3D location of the predetermined reference point of the object, and a 3D location of the other end.

6. The device of claim 1, wherein the 3D movement device moves the ultrasonic transducer module in a direction of at least one of an X-axis, a Y-axis, and a Z-axis.

7. The device of claim 6, wherein the 3D movement device adjusts the location of the ultrasonic transducer module to match a 3D location of an ultrasonic focal point output from the ultrasonic transducer module with a 3D location of a predetermined reference point of an object based on a brain imaging coordinate system for the image registration.

8. The device of claim 7, wherein the 3D movement device adjusts the location of the ultrasonic transducer module to match 3D location coordinates of the ultrasonic focal point output from the ultrasonic transducer module with a 3D location of a targeting point of the object based on the brain imaging coordinate system for the location registration.

9. A driving method of an ultrasonic transducer module control device including an ultrasonic focal point indicator, the method comprising:
measuring an ultrasonic output field characteristic of an ultrasonic transducer module;
manufacturing the ultrasonic focal point indicator based on the ultrasonic output field characteristic;
installing the manufactured ultrasonic focal point indicator in the ultrasonic transducer module so as to display an ultrasonic focal point output from the ultrasonic transducer module; and
adjusting a location of the ultrasonic transducer module for image registration or location registration.

10. The method of claim 9, wherein the adjusting of the location of the ultrasonic transducer module for the image registration includes:
calculating 3D location coordinates of the ultrasonic focal point and 3D location coordinates of a predetermined reference point of an object; and
adjusting a location of the ultrasonic transducer module to match the 3D location coordinates of the ultrasonic focal point with the 3D location coordinates of the predetermined reference point based on a brain imaging coordinate system.

11. The method of claim 10, wherein the adjusting of the location of the ultrasonic transducer module for the location registration further includes:
calculating 3D location coordinates of a targeting point of the object; and
adjusting the location of the ultrasonic transducer module to match the 3D location coordinates of the ultrasonic focal point with the 3D location coordinates of the targeting point based on the brain imaging coordinate system.

12. A non-transitory computer-readable recording medium recorded with a program for executing the driving method of the ultrasonic transducer module control device including the ultrasonic focal point indicator of claim9.
